(19) Europäisches Patentamt
European Patent Office
Office européen des brevets

(11) Numéro de publication : **0 323 303 B1**

# (12) FASCICULE DE BREVET EUROPEEN

(45) Date de publication du fascicule du brevet : **12.08.92 Bulletin 92/33**

(51) Int. Cl.[5] : **C07D 213/82,** A61K 31/455

(21) Numéro de dépôt : **88403182.4**

(22) Date de dépôt : **14.12.88**

(54) **Dérivés de nicotinoyl-pipérazine, procédé de préparation et utilisation en thérapeutique.**

(30) Priorité : **16.12.87 FR 8717563**

(43) Date de publication de la demande : **05.07.89 Bulletin 89/27**

(45) Mention de la délivrance du brevet : **12.08.92 Bulletin 92/33**

(84) Etats contractants désignés : **AT BE CH DE ES FR GB GR IT LI LU NL SE**

(56) Documents cités :
**DE-C- 540 697**
**GB-A- 2 097 790**
**CHEMICAL ABSTRACTS, vol. 108, 1988, page 725, résumé no. 150265c, Columbus, Ohio, US; S. BOTROS et al.: "Synthesis of certainpyridinecarboxamides as potential antihypertensive agents"**

(73) Titulaire : **INSTITUT DE RECHERCHES CHIMIQUES ET BIOLOGIQUES APPLIQUEES (I.R.C.E.B.A.) Société à responsabilité limitée dite:**
**62, Grande-Rue**
**F-78490 Vicq (FR)**

(72) Inventeur : **Danree, Bernard**
**59 bis, Boulevard Deveaux**
**F-78300 Poissy (FR)**
Inventeur : **Faulques, Michelle**
**15, rue Chanzy**
**F-92400 Courbevoie (FR)**
Inventeur : **Lacolle, Jean-Yves**
**27, route des Suisses**
**F-78170 La Celle-Saint-Cloud (FR)**
Inventeur : **Riffaud, Jean-Pierre**
**108, avenue des Etats-Unis**
**F-78000 Versailles (FR)**

(74) Mandataire : **Clisci, Serge et al**
**S.A. FEDIT-LORIOT CONSEILS EN PROPRIETE INDUSTRIELLE 38, avenue Hoche**
**F-75008 Paris (FR)**

**EP 0 323 303 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

### DOMAINE DE L'INVENTION

La présente invention concerne en tant que produits industriels nouveaux des dérivés de nicotinoyl-pipérazine, à savoir plus précisément les composés 4-aryl-1-nicotinoyl-pipérazines de formule I ci-après et leurs sels d'addition. Elle concerne également le procédé de préparation de ces nouveaux composés ainsi que leur utilisation en thérapeutique en tant qu'agents anticonvulsivants.

### ART ANTERIEUR

On sait que l'on a déjà décrit dans le passé un certain nombre de dérivés de pyridinecarboxamides. Voir à cet effet le résumé des Chemical Abstracts 108, 150265c et les documents de brevets allemand DE-C-540 697 et britannique GB-A-2 097 790.

### BUT ET OBJET DE L'INVENTION

Suivant l'un de ses aspects, la présente invention se propose de fournir de nouveaux composés appartenant à la famille des nicotinoyl-pipérazines, structurellement différents des dérivés de l'art antérieur précité et qui soient utiles en thérapeutique eu égard à leurs propriétés anticonvulsivantes bénéfiques.

Suivant un autre aspect de l'invention on préconise un procédé de préparation approprié à la synthèse desdits dérivés.

Suivant encore un autre aspect de l'invention on propose une composition thérapeutique renfermant comme ingrédient actif au moins un desdits dérivés.

Les nouveaux dérivés selon l'invention qui appartiennent à la famille des nicotinoyl-pipérazines sont caractérisés en ce qu'ils sont choisis parmi l'ensemble comprenant :

(i) les 4-aryl-1-nicotinoyl-pipérazines de formule

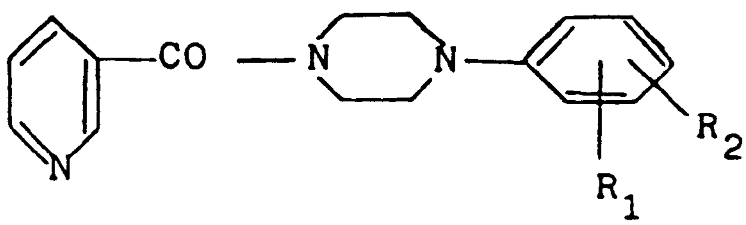

(I)

dans laquelle $R_1$ et $R_2$, qui peuvent être identiques ou différents, représentent chacun l'atome d'hydrogène, un atome d'halogène (notamment F, Cl, Br), un groupe $CF_3$, alkyl en $C_1$-$C_4$, alkoxy en $C_1$-$C_4$ ou acyle en $C_2$-$C_4$; et,

(ii) leurs sels d'addition.

### DESCRIPTION DETAILLEE DE L'INVENTION

L'invention concerne donc en tant que produits industriels nouveaux les composés de formule I et leurs sels d'addition.

Dans la définition de $R_1$ et $R_2$, par atomes d'halogène, on entend ici les atomes de fluor, de chlore et de brome, les atomes d'halogène préférés pour $R_1$ et/ou $R_2$ sont le chlore et le fluor, le plus intéressant sur le plan thérapeutique étant le chlore.

Par groupe alkyle en $C_1$-$C_4$ et alkoxy en $C_1$-$C_4$ on entend ici des groupes comprenant une chaîne hydrocarbonée saturée ayant de 1 à 4 atomes de carbone et qui soit linéaire ou ramifiée.

Par groupe acyle en $C_2$-$C_4$ on entend un groupe comprenant un reste CO lié à un reste alkyle en $C_1$-$C_3$. Le groupe acyle préféré est ici le groupe $COCH_3$.

D'un point de vue pratique on préfère, eu égard à l'efficacité thérapeutique, que $R_1$ et $R_2$ ne représentent pas tous deux, l'atome d'hydrogène. De façon avantageuse $R_1$ et $R_2$, qui peuvent être identiques ou différents, représenteront chacun Cl, F, $CF_3$, $CH_3$, $C_2H_5$, $OCH_3$, $OC_2H_5$ ou $COCH_3$, un seul des $R_1$ et $R_2$ pouvant représenter, le cas échéant, l'atome d'hydrogène.

De façon préférée, le groupe $R_1$ sera situé en position 2 ou ortho du noyau benzénique, et le groupe $R_2$

sur l'une quelconque des autres positions libres 3,4,5,6, notamment en position méta ou para.

Par sels d'addition, on entend ici les sels d'addition d'acide obtenus par réaction d'une base libre de formule I avec un acide minéral ou organique, d'une part, et les sels d'ammonium, d'autre part. Parmi les acides utilisables pour salifier les bases libres de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale, les sels d'addition d'acide sont préférés aux sels d'ammonium, et, parmi lesdits sels d'addition d'acide, les chlorhydrates des composés de formule I sont les substances préférées pour utilisation thérapeutique en tant que moyens anticonvulsivants.

Les composés de formule I peuvent être préparés selon une méthode connue en soi par application de mécanismes réactionnels classiques. Le procédé que l'on préconise suivant l'invention consiste à faire réagir une N-aryl-pipérazine de formule

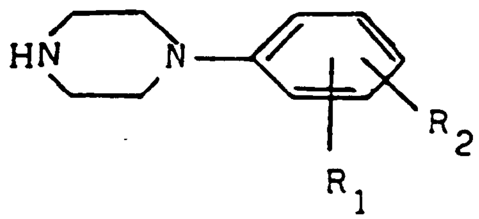

(II)

où $R_1$ et $R_2$ sont définis comme indiqué ci-dessus, avec un halogénure d'acide nicotinique de formule

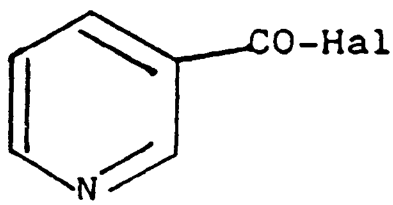

(III)

où Hal représente un atome d'allogène notamment F, Cl, Br ou I. Sur le plan de la synthèse l'atome d'halogène préféré est le chlore.

Cette réaction est avantageusement effectuée à la température de reflux du milieu réactionnel pendant au moins 0,25 h, dans des conditions sensiblement stoechiométriques (c'est-à-dire à raison de 1 mole de II pour environ 1 mole de III), au sein d'un solvant approprié. Un des solvants appropriés que l'on préconise ici est la pyridine.

On a consigné de façon non limitative dans le tableau I ci-après un certain nombre de composés typiques selon l'invention. Les points de fusion qui ont été mentionnés dans ce tableau sont des points de fusion instantanée déterminés au banc Kofler. Dans ledit tableau, Me désigne le groupe méthyle, Et le groupe éthyle, Ac le groupe acétyle, et AcOEt l'acétate d'éthyle, d'une part, et par commodité le groupe $R_1$ est placé en position ortho et le groupe $R_2$ sur les autres positions du noyau benzénique, d'autre part.

**TABLEAU I**

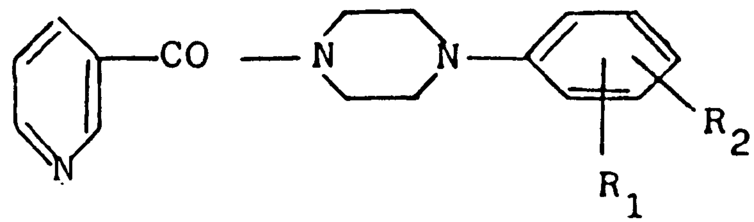

(I)

| Produit | No. de Code | $R_1$ | $R_2$ | F (°C) |
|---|---|---|---|---|
| Ex 1 (a) | B-1370 | H | 3-$CF_3$ | 238-240 (d) |
| Ex 2 (a) | B-1371 | H | 3-Cl | 153-155 |
| Ex 3 (a) | B-1372 | 2-Me | 3-Me | 148-150 (e) |
| Ex 4 (b) | B-1373 | 2-Cl | H | 98-100 (f) |
| Ex 5 (c) | B-1374 | 2-OEt | H | 88-90 (g) |
| Ex 6 (c) | B-1230 | H | 3-$CF_3$ | 77-80 (g) |
| Ex 7 (b) | B-1368 | H | 4-$CF_3$ | 143-145 (h) |
| Ex 8 (c) | B-1273 | 2-F | H | 69-70 (i) |
| Ex 9 (a) | B-1252 | 2-F | H | 150-152 (j) |
| Ex 10 (c) | B-1247 | H | 4-F | 115-116 (k) |
| Ex 11 (a) | B-1251 | H | 4-F | 195-196 (e) |
| Ex 12 (b) | B-1363 | 2-$CF_3$ | H | 128-132 (f) |
| Ex 13 (c) | B-1275 | 2-Me | H | 134-135 (g) |
| Ex 14 (a) | B-1282 | 2-Me | H | 148-150 (h) |

**TABLEAU I (suite 1)**

| Produit | No. de Code | $R_1$ | $R_2$ | F (°C) |
|---|---|---|---|---|
| Ex 15 (a) | B-1274 | H | 3-OMe | 174-175 (1) |
| Ex 16 (c) | B-1280 | H | 4-OMe | 95-96 (m) |
| Ex 17 (a) | B-1295 | H | 4-OMe | 190-192 (1) |
| Ex 18 (c) | B-1276 | H | 3-OEt | 106-108 (n) |
| Ex 19 (a) | B-1286 | H | 3-OEt | 184-185 (e) |
| Ex 20 (c) | B-1271 | H | 4-Ac | 163-164 (o) |
| Ex 21 (b) | B-1281 | H | 4-Ac | 178-180 (e) |
| Ex 22 (c) | B-1279 | 2-Me | 4-Cl | 113-114 (m) |
| Ex 23 (b) | B-1294 | 2-Me | 4-Cl | 165-170 (1) |
| Ex 24 (c) | B-1303 | 2-Me | 5-Cl | <50 (p) |
| Ex 25 (a) | B-1248 | 2-Me | 5-Cl | 180-182 |
| Ex 26 (b) | B-1352 | 2-Me | 6-Cl | 178-180 (j) |
| Ex 27 (c) | B-1277 | H | H | 65-66 |
| Ex 28 (a) | B-1254 | H | H | 210-212 (1) |
| Ex 29 (a) | B-1383 | 2-OEt | H | 184-185 (j) |
| Ex 30 (b) | B-1382 | 2-Me | 6-Me | 178-180°C (j) |

Notes

(a) : dichlorhydrate ;
(b) : monochlorhydrate ;
(c) : base libre ;
(d) : solvant de recristallisation : MeOH

**TABLEAU I (fin)**

Notes (suite et fin)

(e) : solvant de recristallisation : EtOH
(f) : solvant de recristallisation : mélange AcOEt/isopropanol (1/1)v/v
(g) : solvant de recristallisation : AcOEt
(h) : solvant de recristallisation : isopropanol
(i) : solvant de recristallisation : éther de pétrole (fraction ayant un point d'ébullition de 40-65°C sous pression normale)
(j) : solvant de recristallisation : mélange AcOEt/EtOH (1/1)v/v
(k) : solvant de recristallisation : mélange AcOEt/pentane (1/1)v/v
(l) : solvant de recristallisation : mélange isopropanol/MeOH (1/1)v/v
(m) : solvant de recristallisation : mélange AcOEt/hexane (1/1)v/v
(n) : solvant de recristallisation : mélange AcOEt/cyclohexane (1/1)v/v
(o) : solvant de recristallisation : mélange AcOEt/MeOH (1/1)v/v
(p) : solvant de recristallisation : mélange $CH_2Cl_2$/MeOH (1/1)v/v

Le meilleur mode de mise en oeuvre de l'invention consiste à faire appel à la 1-nicotinoyl-4-(3-trifluorométhylphényl)-pipérazine, à la 4-(3-chlorophényl)-1-nicotinoyl-pipérazine, à la 4-(4-fluorophényl)-1-nicotinoyl-pipérazine, à la 4-(4-chloro-2-méthylphényl)-1-nicotinoyl-pipérazine, à la 4-(5-chloro-2-méthylphényl)-1-nicotinoyl-pipérazine et à leurs sels d'addition. Ces produits sont les plus intéressants en thérapeutique eu égard notamment à leurs propriétés anticonvulsivantes. Parmi ceux-ci, les plus efficaces sur le plan thérapeutique sont la 1-nicotinoyl-4-(3-trifluorométhylphényl)-pipérazine (Ex 6 ; No de Code : B-1230) et son dichlorhydrate (Ex 1 ; No de Code : B-1370).

Les composés de formule I suivant l'invention et leurs sels d'addition sont utiles en thérapeutique. Ils agissent en tant qu'ingrédients actifs anticonvulsivants et sont donc recommandés dans le traitement des convulsions et de l'épilepsie chez l'homme.

Suivant l'invention, on préconise donc une composition thérapeutique, caractérisée en ce qu'elle renferme, en association avec un excipient physiologiquement acceptable, au moins un composé choisi parmi les 4-aryl-1-nicotinoyl-pipérazines de formule I et leurs sels d'addition non-toxiques.

Bien entendu dans une telle composition le principe actif intervient en quantité thérapeutiquement efficace.

Suivant l'invention, on préconise une utilisation d'une substance appartenant à l'ensemble des 4-aryl-1-nicotinoyl-pipérazines de formule I et de leurs sels d'addition non-toxiques pour l'obtention d'un médicament destiné à une utilisation en thérapeutique vis-à-vis des convulsions et notamment des convulsions d'origine épileptique.

**PREPARATION I**

Obtention de la 1-nicotinoyl-4-(3-trifluorométhylphényl)-pipérazine

(Exemple 6 ; No de Code : B-1230).

Dans un tricol de deux litres muni d'un réfrigérant avec compte-bulles à $H_2SO_4$, d'un thermomètre et d'une agitation pneumatique, on introduit 600 ml de pyridine et 207,2 g (0,9 mole) de 1-(3-trifluorométhylphényl)-pipérazine. Au mélange résultant on ajoute par portions 127,5 g (0.9 mole) de chlorure d'acide nicotinique. On porte le milieu réactionnel au reflux pendant une heure. Après refroidissement jusqu'à température ambiante (15-20°C), on verse sur trois litres d'eau glacée sous agitation. On extrait la phase aqueuse par 3 x 300 ml de chlorure de méthylène. Les phases organiques sont rassemblées, lavées par 2 x 250 ml de solution de $NaHCO_3$ à 5 % (p/v), puis par 3 x 250 ml d'eau jusqu'à neutralité. On sèche sur sulfate de sodium, filtre, recueille le filtrat et chasse le solvant sous vide. On obtient 287,3 g (rendement = 95,2 %) d'un liquide brun très épais, qui présente une pureté de 99 % en chromatographie en phase vapeur (C.P.V.), et deux tâches (1 spot principal + léger spot secondaire) en chromatographie sur couche mince (C.C.M.) avec comme phase mobile le mélange $CH_2Cl_2$-$CH_3OH$ (80/20) v/v.

Ce produit brut ainsi obtenu est purifié par distillation fractionnée sous vide. On recueille 247,8 g (rendement global : 82,1 %) du produit attendu que se présente sous la forme d'une huile orangée.

$Eb_{0,3\ mmHg}$ = 225-230°C
(0,3 mmHg correspond approximativement à 39,9 Pa).
Pureté C.P.V. > 99 %
C.C.M. : monotache [$CH_2Cl_2$-$CH_3OH$(80/20)v/v]
spectre I.R. : conforme à la structure proposée
spectre R.M.N. : conforme à la structure proposée

Le produit huileux ainsi obtenu est cristallisé par addition d'éther. Par recristallisation dans l'acétate d'éthyle des cristaux ainsi obtenus, on obtient le composé B-1230 purifié :

$F_{inst}$ = 77-80°C.

**PREPARATION II**

Obtention du dichlorhydrate de 1-nicotinoyl-4-(3-trifluorométhylphényl)-pipérazine.

(Exemple 1 ; No de Code : B-1370).

On dissout 247,8 g (0.74 mole) de 1-nicotinoyl-4-(3-trifluorométhylphényl)-pipérazine, qui a été obtenue comme indiqué à la préparation I, dans trois litres de diéthyléther anhydre. La solution résultante refroidie est saturée par un courant de HCl sec. Les cristaux formés sont filtrés (notamment sur verre fritté), lavés avec du diéthyléther anhydre puis séchés sous vide à 40°C. Après recristallisation de ces cristaux dans le méthanol, on obtient 214,2 g (rendement : 70,9 %) de B-1370 sous la forme de cristaux blancs.

$F_{inst}$ = 238 - 240°C

C.C.M. = monotache [$CH_2Cl_2$-$CH_3OH$-$NH_4OH$ (80/20/0,5) v/v]
Pureté C.P.V. > 99 %
Spectre I.R. : conforme à la structure proposée
Spectre R.M.N. : conforme à la structure proposée

## ESSAIS PHARMACOLOGIQUES

On a résumé ci-après un certain nombre d'essais qui ont été entrepris avec les composés suivant l'invention par comparaison avec deux produits anticonvulsivants de référence, à savoir le valproate de sodium (en abrégé VALP) et la triméthadione (en abrégé TRIM).

### I - TOXICITE

La toxicité des produits à étudier a été recherchée par administration orale, à l'aide d'une sonde oesophagienne, de chacun desdits produits chez la souris mâle d'un poids corporel de 18 à 22 g et répartie en lot de 10 à 20 animaux chacun. La mortalité a été enregistrée pendant une période de 14 jours.

Les résultats qui ont été obtenus et qui sont exprimés sous forme de DL-50 en mg/kg sont consignés dans le tableau II ci-après.

### II - PROPRIETES ANTICONVULSIVANTES

Les propriétés anticonvulsivantes ont été recherchées chez la souris mâle trente minutes après administration orale des substances à étudier, par induction de crises convulsives.

Ces crises sont provoquées, soit par injection intrapéritonéale de pentétrazol (125 mg/kg) soit par stimulation électrique de la cornée (électrochoc supra-maximal ; en abrégé : E.C.M.).

Les résultats exprimés sous forme de dose efficace 50 (DE-50) per os, (dose protégeant 50 % des animaux), sont consignés dans le tableau III ci-après. Ils montrent que les produits suivant l'invention exercent un effet protecteur vis-à-vis des convulsions au moins aussi important que celui des deux produits de référence (VALP et TRIM) quand on considère les valeurs des DE-50.

## TABLEAU II

## TOXICITE PER OS CHEZ LA SOURIS*

| Produit | No. de Code | DL-50 (mg/kg) |
|---|---|---|
| Ex 1 | B-1370 | 575 |
| Ex 2 | B-1371 | 501 |
| Ex 3 | B-1372 | >1000 |
| Ex 4 | B-1373 | >1000 |
| Ex 5 | B-1374 | 1000 |
| Ex 6 | B-1230 | 422 |
| Ex 7 | B-1368 | >1000 |
| Ex 8 | B-1273 | .1000 |
| Ex 9 | B-1252 | >1000 |
| Ex 10 | B-1247 | 600 |
| Ex 11 | B-1251 | 625 |
| Ex 12 | B-1363 | >1000 |
| Ex 13 | B-1275 | 1000 |
| Ex 14 | B-1282 | >1000 |
| Ex 15 | B-1274 | >1000 |
| Ex 16 | B-1280 | >1000 |
| Ex 17 | B-1295 | >1000 |
| Ex 18 | B-1276 | 540 |
| Ex 19 | B-1286 | 1000 |
| Ex 20 | B-1271 | >1000 |
| Ex 21 | B-1281 | >1000 |
| Ex 22 | B-1279 | 540 |
| Ex 23 | B-1294 | >1000 |
| Ex 24 | B-1303 | >1000 |
| Ex 25 | B-1248 | >1000 |
| Ex 26 | B-1352 | >1000 |
| Ex 27 | B-1277 | >1000 |
| Ex 28 | B-1254 | >1000 |
| Ex 29 | B-1383 | ND |
| Ex 30 | B-1382 | >1000 |
| VALP | - | 977 |
| TRIM | - | 2182 |

Note

* Toxicité déterminée sur des lots de 10 à 20 souris mâles chacun.

ND : Non déterminée.

TABLEAU III
PROPRIETES ANTICONVULSIVANTES CHEZ LA SOURIS*

| Produit | No. de Code | DE-50 vis-à-vis des convulsions induites par : PTZ (mg/kg) | E.C.M. (mg/kg) |
|---|---|---|---|
| Ex 1 | B-1370 | 17 | 19 |
| Ex 2 | B-1371 | 50 | 78 |
| Ex 3 | B-1372 | 110 | >200 |
| Ex 4 | B-1373 | 160 | 200 |
| Ex 5 | B-1374 | >200 | >200 |
| Ex 6 | B-1230 | 13 | 20 |
| Ex 7 | B-1368 | >200 | >200 |
| Ex 8 | B-1273 | 180 | 180 |
| Ex 9 | B-1252 | >200 | >200 |
| Ex 10 | B-1247 | 62 | 75 |
| Ex 11 | B-1251 | 68 | 75 |
| Ex 12 | B-1363 | >200 | >200 |
| Ex 13 | B-1275 | 161 | 200 |
| Ex 14 | B-1282 | 107 | >200 |
| Ex 15 | B-1274 | 183 | >200 |
| Ex 16 | B-1280 | 120 | >200 |
| Ex 17 | B-1295 | 200 | >200 |
| Ex 18 | B-1276 | 72 | 118 |
| Ex 19 | B-1286 | 142 | 150 |
| Ex 20 | B-1271 | >200 | >200 |
| Ex 21 | B-1281 | >200 | >200 |
| Ex 22 | B-1279 | 55 | 87 |
| Ex 23 | B-1294 | 124 | >200 |
| Ex 24 | B-1303 | 63 | 83 |
| Ex 25 | B-1248 | 79 | 87 |
| Ex 26 | B-1352 | >200 | >200 |
| Ex 27 | B-1277 | 156 | >200 |
| Ex 28 | B-1254 | >200 | >200 |
| Ex 29 | B-1383 | ND | ND |
| Ex 30 | B-1382 | 138 | ND |
| VALP | - | 244 | 242 |
| TRIM | - | 251 | 500 |

Notes

PTZ : pentétrazol ;
E.C.M. : électrochoc supra-maximal ;
* : DE-50 en mg/kg déterminées per os sur des lots de dix souris mâles chacun.
ND : Non déterminée.

## III- PROPRIETES NEUROTOXIQUES

Les propriétés neurotoxiques ont été appréciées chez la souris mâle suivant le test dit de "la tige tournante" ("Rota-rod") effectué trente minutes après administration orale des substances à étudier. On observe la chute des animaux (répartis en lots de dix souris mâles chacun par dose et par produit) au cours des deux minutes qui suivent leur installation sur la tige.

Les résultats, qui ont été obtenus, sont exprimés sous la forme de dose neurotoxique 50 (DT-50) par os (dose provoquant la chute de 50 % des animaux et exprimée en mg/kg). Ces résultats sont consignés dans le tableau IV ci-après, dans lequel, pour comparaison avec les produits de référence précités (VALP et TRIM), on a également donné les valeurs des rapports DT-50/DE-50 (index de protection) et DL-50/DE-50 (index thérapeutique) dans lesquels les DE-50 sont les doses efficaces 50 vis-à-vis des convulsions induites par le pentétrazol (PTZ) et l'électrochoc supra-maximal (E.C.M.) déterminées comme indiqué ci-dessus (voir notamment le tableau III).

Les résultats du tableau IV montrent clairement que les composés suivant l'invention Ex 1 (B-1370) et Ex 6 (B-1230) présentent notamment des index de protection et des index thérapeutiques meilleurs que ceux des produits de référence, le valproate de sodium et la triméthadione.

**TABLEAU IV**

**ACTIVITE NEUROTOXIQUE**

| Produit | No de Code | DT-50* (mg/kg) | Index de protection PTZ | Index de protection E.C.M. | DL-50 (mg/kg) | Index thérapeutique PTZ | Index thérapeutique E.C.M. |
|---|---|---|---|---|---|---|---|
| Ex 1 | B-1370 | 154 | 9,1 | 8,1 | 575 | 33,8 | 30,2 |
| Ex 6 | B-1230 | 119 | 9,1 | 6,0 | 422 | 32,5 | 21,1 |
| Ex 8 | B-1273 | 500 | 2,8 | 2,8 | 1000 | 5,6 | 5,6 |
| Ex 13 | B-1275 | 800 | 4,9 | 4,0 | 1000 | 6,2 | 5,0 |
| Ex 14 | B-1282 | 635 | 5,9 | <3,0 | >1000 | >10,0 | <5,0 |
| Ex 18 | B-1276 | 291 | 4,0 | 2,5 | 540 | 7,5 | 4,6 |
| Ex 19 | B-1286 | 250 | 1,8 | 1,7 | 1000 | 7,0 | 6,7 |
| Ex 22 | B-1279 | 300 | 5,4 | 3,4 | 540 | 9,8 | 6,2 |
| Ex 23 | B-1294 | 650 | 7,4 | 4,5 | >1000 | > 8,1 | <5,0 |
| Ex 24 | B-1303 | 525 | 8,3 | 6,3 | >1000 | >15,9 | >12,0 |
| Ex 25 | B-1248 | 355 | 4,5 | 4,1 | >1000 | >12,7 | >11,5 |
| VALP | - | 670 | 2,7 | 2,8 | 977 | 4,0 | 4,0 |
| TRIM | - | 1000 | 4,0 | 2,0 | 2182 | 8,7 | 4,4 |

Notes

Index de protection : DT-50 per os/DE-50 per os
Index thérapeutique : DL-50 per os/DE-50 per os
* dose déterminée per os sur des lots de dix souris mâles chacun.

## Revendications

### Revendications pour les Etats contractants suivants : AT, BE, CH, DE, FR, GB, IT, LI, LU, NL, SE

1. Composé appartenant à la famille des nicotinoyl-pipérazines, caractérisé en ce qu'il est choisi parmi l'ensemble comprenant:

(i) les 4-aryl-1-nicotinoyl-pipérazines de formule

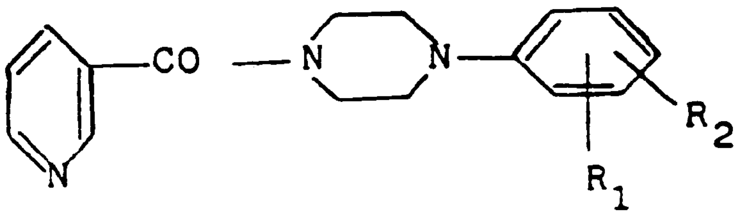

(I)

dans laquelle $R_1$ et $R_2$, qui peuvent être identiques ou différents, représentent chacun l'atome hydrogène, un atome d'halogène, un groupe $CF_3$, alkyle en $C_1$-$C_4$, alkoxy en $C_1$-$C_4$ ou acyle en $C_2$-$C_4$ ; et,

(ii) leurs sels d'addition.

2. Composé suivant la revendication 1, caractérisé en ce que l'atome d'halogène intervenant dans la définition de $R_1$ et $R_2$ est choisi parmi l'ensemble constitué par F, Cl et Br.

3. Composé suivant la revendication 1, caractérisé en ce que $R_1$ et $R_2$, qui peuvent être identiques ou différents, représentent chacun Cl, F, $CF_3$, $CH_3$, $C_2H_5$, $OCH_3$, $OC_2H_5$ ou $COCH_3$, un seul des $R_1$ et $R_2$ pouvant représenter, le cas échéant, l'atome d'hydrogène.

4. Composé suivant l'une quelconque des revendications 1 et 3, caractérisé en ce que $R_1$ est en position 2 et $R_2$ en position 3,4,5 ou 6 du noyau benzénique.

5. 1-Nicotinoyl-4-(3-trifluorométhylphényl)-pipérazine et ses sels d'addition.

6. 4-(3-Chlorophényl)-1-nicotinoyl-pipérazine et ses sels d'addition.

7. 4-(4-Fluorophényl)-1-nicotinoyl-pipérazine et ses sels d'addition.

8. 4-(4-Chloro-2-méthylphényl)-1-nicotinoyl-pipérazine et ses sels d'addition.

9. 4-(5-Chloro-2-méthylphényl)-1-nicotinoyl-pipérazine et ses sels d'addition.

10. 4-(4-Acétylphényl)-1-nicotinoyl-pipérazine et ses sels d'addition.

11. Procédé de préparation d'un composé 4-aryl-1-nicotinoyl-pipérazine de formule I selon la revendication 1 et de ses sels d'addition, ledit procédé étant caractérisé en ce que l'on fait réagir une N-aryl-pipérazine de formule

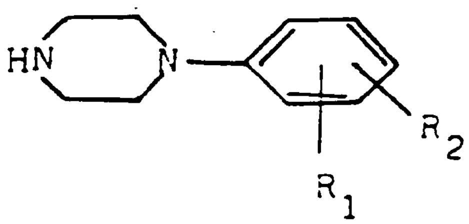

(II)

où $R_1$et $R_2$ sont définis comme indiqué ci-dessus, avec une halogénure d'acide nicotinique de formule

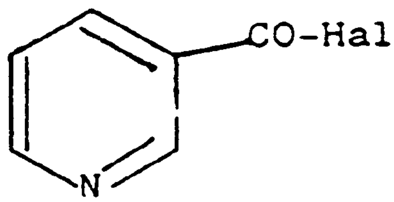

(III)

où Hal représente F, Cl, Br ou I, l'atome d'halogène préféré étant le chlore.

12. Procédé suivant la revendication 11, caractérisé en ce que l'on fait réagir 1 mole de N-aryl-pipérazine de formule II avec environ 1 mole d'halogénure d'acide nicotinique de formule III pendant au moins 0,25 h à la température de reflux du milieu réactionnel.

13. Composition thérapeutique, caractérisée en ce qu'elle renferme, en association avec un excipient physiologiquement acceptable, au moins un composé choisi parmi les 4-aryl-1-nicotinoyl-pipérazines de formule I suivant la revendication 1 et leurs sels d'addition non-toxiques.

14. Utilisation d'une substance appartenant à l'ensemble des 4-aryl-1-nicotinoyl-pipérazines de formule I suivant la revendication 1 et de leurs sels d'addition non-toxiques pour l'obtention d'un médicament destiné à une utilisation en thérapeutique vis-à-vis des convulsions et notamment des convulsions d'origine épileptique.

**Revendications pour les Etats contractants suivants : ES, GR**

1. Procédé de préparation d'un composé nicotinoyl-piperazine choisi parmi l'ensemble comprenant :
(i) les 4-aryl-nicotinoyl-pipérazines de formule

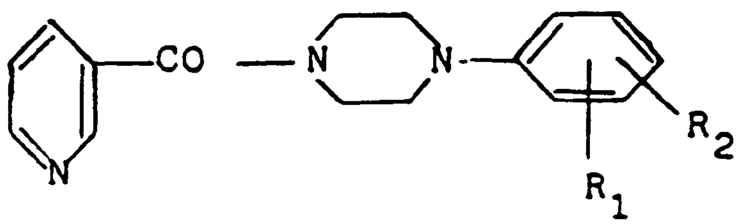

(I)

dans laquelle $R_1$ et $R_2$, qui peuvent être identiques ou différents, représentent chacun l'atome d'hydrogène, un atome d'halogène, un groupe $CF_3$, alkyle en $C_1$-$C_4$, alkoxy en $C_1$-$C_4$ ou acyle en $C_2$-$C_4$ ; et
(ii) leurs sels d'addition ; ledit procédé étant caractérisé en ce que l'on fait réagir une N-aryl-piperazine de formule

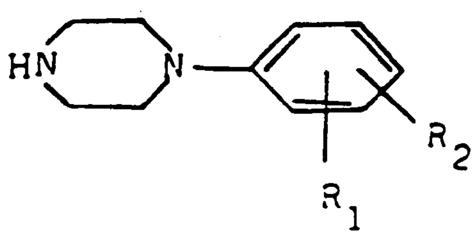

(II)

où $R_1$ et $R_2$ sont définis comme indiqué ci-dessus, avec un halogénure d'acide nicotinique de formule

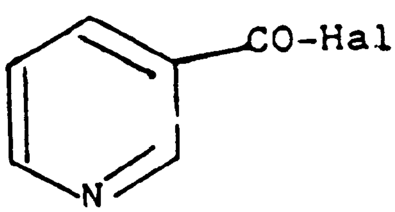

(III)

où Hal représente F, Cl, Br ou I.

2. Procédé suivant la revendication 1, caractérisé en ce que l'on fait réagir 1 mole de N-aryl-piperazine de formule II avec environ 1 mole d'halogénure d'acide nicotinique de formule III pendant au moins 0,25 h à la température de reflux du milieu réactionnel.

3. Procédé suivant la revendication 1, caractérisé en ce que Hal est Cl.

4. Procédé suivant la revendication 1, caractérisé en ce que l'atome d'halogène intervenant dans la définition de $R_1$ et $R_2$ est choisi parmi l'ensemble constitué par F, Cl, et Br.

5. Procédé suivant la revendication 1, caractérisé en ce que $R_1$ et $R_2$, qui peuvent être identiques ou différents, représentent chacun Cl, F, $CF_3$, $CH_3$, $C_2H_5$, $OCH_3$, $OC_2H_5$ or $COCH_3$, un seul des $R_1$ et $R_2$ pouvant représenter, le cas échéant, l'atome d'hydrogène.

6. Procédé suivant l'une quelconque des revendications 1 et 5, caractérisé en ce que $R_1$ est en position 2 et $R_2$ en position 3, 4, 5 ou 6 du noyau benzénique.

## Patentansprüche

### Patentansprüche für folgende Vertragsstaaten : AT, BE, CH, DE, FR, GB, IT, LI, LU, NL, SE

1. Zur Substanzgruppe der Nicotinoyl-piperazine gehörende Verbindungen, dadurch gekennzeichnet, daß sie ausgewählt sind aus der Gruppe

(i) der 4-Aryl-1-nicotinoyl-piperazine gemäß der Formel

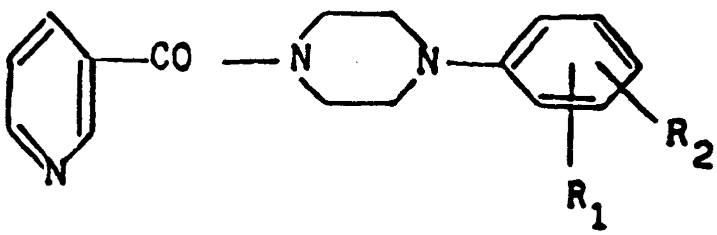

(I)

in der $R_1$ und $R_2$, die identisch oder verschieden sein können, ein Wasserstoffatom, ein Halogenatom, die Gruppe $CF_3$, eine $C_1$-$C_4$-Alkylgruppe, eine $C_1$-$C_4$-Alkoxygruppe oder einen $C_2$-$C_4$-Acylrest darstellen, und

(ii) den Additionssalzen derselben.

2. Verbindungen gemäß Anspruch 1, dadurch gekennzeichnet, daß das Halogenatom bei der Definition von $R_1$ und $R_2$ ausgewählt ist aus F, Cl und Br.

3. Verbindungen gemäß Anspruch 1, dadurch gekennzeichnet, daß $R_1$ und $R_2$, die identisch oder verschieden sein können, Cl, F, $CF_3$, $CH_3$, $C_2H_5$, $OCH_3$, $OC_2H_5$ oder $COCH_3$ bedeuten und allenfalls einer der Reste $R_1$ und $R_2$ ein Wasserstoffatom ist.

4. Verbindungen gemäß einem der Ansprüche 1 und 3, dadurch **gekennzeichnet**, daß der Rest $R_1$ sich in der Position 2 befindet und der Rest $R_2$ in der Position 3, 4, 5 oder 6 vom Benzolring angeordnet ist.

5. 1-Nicotinoyl-4-(3-trifluormethylphenyl)-piperazin und dessen Additionssalze.

6. 4-(3-Chlorphenyl)-1-nicotinoyl-piperazin und dessen Additionssalze.

7. 4-(4-Fluorphenyl)-1-nicotinoyl-piperazin und dessen Additionssalze.

8. 4-(4-Chlor-2-methylphenyl)-1-nicotinoyl-piperazin und dessen Additionssalze.

9. 4-(5-Chlor-2-methylphenyl)-1-nicotinoyl-piperazin und dessen Additionssalze.

10. 4-(4-Acetylphenyl)-1-nicotinoyl-piperazin und dessen Additionssalze.

11. Verfahren zur Herstellung von 4-Aryl-1-nicotinoyl-piperazin-Verbindungen gemäß der allgemeinen Formel I und deren Additionssalzen, dadurch gekennzeichnet, daß man ein N-Aryl-piperazin der Formel

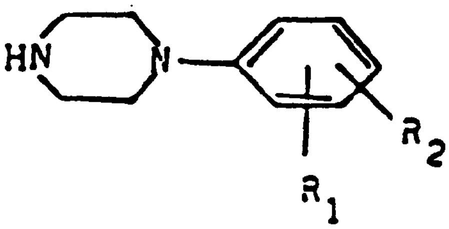

(II)

in der $R_1$ und $R_2$ der oben angeführten Definition entsprechen, mit einem Nicotinsäurehalogenid der Formel

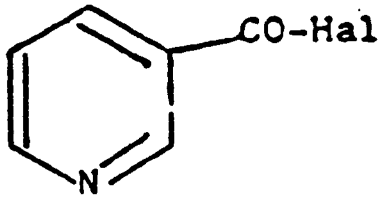

(III)

in der Hal F, Cl, Br oder J entspricht und Chlor das bevorzugte Halogenatom ist, zur Reaktion bringt.

12. Verfahren gemäß Anspruch 11, dadurch gekennzeichnet, daß man 1 Mol des N-Arylpiperazins gemäß der Formel II mit etwa 1 Mol des Nicotinsäurehalogenids gemäß der Formel III während mindestens 0,25 h unter Rückflußtemperatur der Reaktionsmischung umsetzt.

13. Therapeutische Zusammensetzung, dadurch gekennzeichnet, daß sie zusammen mit einem physiologisch annehmbaren Träger mindestens eine Verbindung enthält, die ausgewählt ist aus den 4-Aryl-1-nicotinoyl-piperazinen gemäß der im Anspruch 1 angeführten Formel I und deren Additionssalzen.

14. Verwendung einer Verbindung aus der Gruppe der 4-Aryl-1-nicotinoyl-piperazine gemäß der im Anspruch 1 angeführten Formel I und deren nicht-toxischen Additionssalzen zur Gewinnung eines Medikaments, das bestimmt ist für den therapeutischen Einsatz im Falle von Krämpfen und insbesondere von Krämpfen epileptischen Ursprungs.

**Patentansprüche für folgende Vertragsstaaten : ES, GR**

1. Verfahren zur Herstellung von Nicotinoyl-piperazinen, ausgewählt aus der Gruppe, die gebildet wird von:
(i) 4-Aryl-1-nicotinoyl-piperazine gemäß der Formel

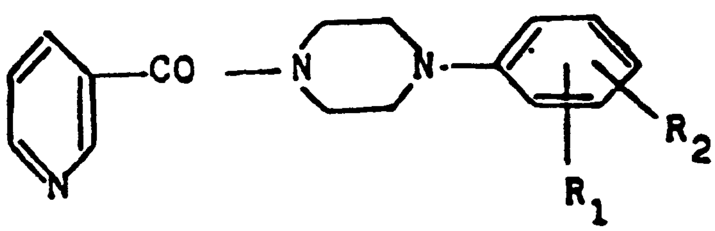

(I)

in der $R_1$ und $R_2$, die identisch oder verschieden sein können, ein Wasserstoffatom, ein Halogenatom, die Gruppe $CF_3$, eine $C_1$-$C_4$-Alkylgruppe, eine $C_1$-$C_4$-Alkoxygruppe oder einen $C_2$-$C_4$-Acylrest darstellen, und
(ii) den Additionssalzen dergelben, dadurch gekennzeichnet, daß man ein N-Aryl-piperazin der Formel

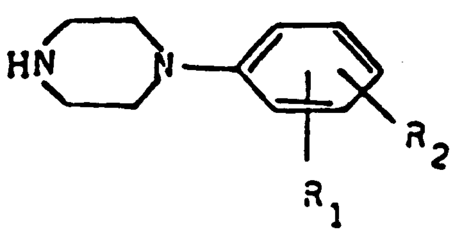

(II)

in der $R_1$ und $R_2$ der oben angeführten Definition entsprechen, mit einem Nicotinsäurehalogenid der Formel

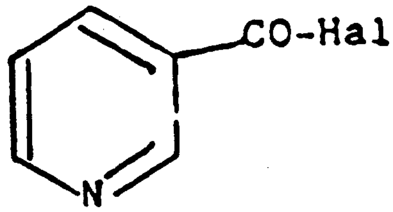

(III)

in der Hal F, Cl, Br oder J entspricht, zur Reaktion bringt.

2. Verfahren gemäß Anspruch 1, dadurch gekennzeichnet, daß man 1 Mol des N-Arylpiperazins gemäß der Formel II mit etwa 1 Mol des Nicotinsäurehalogenids gemäß der Formel III während mindestens 0,25 h unter Rückflußtemperatur der Reaktionsmischung umsetzt.

3. Verfahren gemäß Anspruch 1, dadurch gekennzeichnet, daß Hal Cl bedeutet.

4. Verfahren gemäß Anspruch 1, dadurch gekennzeichnet, daß das Halogenatom in der Definition für $R_1$ und $R_2$ ausgewählt ist aus der von F, Cl und Br gebildeten Gruppe.

5. Verfahren gemäß Anspruch 1, dadurch gekennzeichnet, daß $R_1$ und $R_2$, die identisch oder verschieden sein können, Cl, F, $CF_3$, $CH_3$, $C_2H_5$, $OCH_3$, $OC_2H_5$ oder $COCH_3$ bedeuten und allenfalls einer der Reste $R_1$ und $R_2$ ein Wasserstoffatom ist.

6. Verfahren gemäß einem der Ansprüche 1 und 5, dadurch gekennzeichnet, daß der Rest $R_1$ sich in der Position 2 befindet und der Rest $R_2$ in der Position 3, 4, 5 oder 6 vom Benzolring angeordnet ist.

## Claims

**Claims for the following Contracting States : AT, BE, CH, DE, FR, GB, IT, LU, NL, SE**

1. A compound belonging to the family of the nicotinoyl-piperazines, which is selected from the group consisting of :

(i) 4-aryl-1-nicotinoylpiperazines of the formula

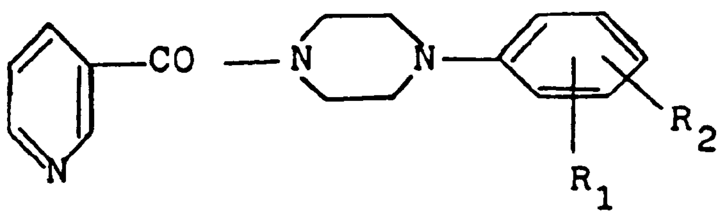

(I)

in which $R_1$ and $R_2$, which can be identical or different, each represent the hydrogen atom, a halogen atom or a $CF_3$, $C_1$-$C_4$-alkyl or $C_1$-$C_4$-alkoxy group ; and

(ii) addition salts thereof.

2. A compound according to claim 1 wherein the halogen atom within the definition of $R_1$ and $R_2$ is selected from F, Cl and Br.

3. A compound according to claim 1 wherein $R_1$ and $R_2$, which can be identical or different, each represent Cl, F, $CF_3$, $CH_3$, $C_2H_5$, $OCH_3$ or $OC_2H_5$, it being possible, if appropriate, for only one of the groups $R_1$ and $R_2$ to represent the hydrogen atom.

4. A compound according to claim 1 or claim 3 wherein $R_1$ is in the 2-position and $R_2$ is in the 3-4-5-or 6-position of the benzene ring.

5. 1-Nicotinoyl-4-(3-trifluoromethylphenyl)piperazine and addition salts thereof.

6. 4-(3-Chlorophenyl)-1-nicotinoylpiperazine and addition salts thereof.

7. 4-(4-Fluorophenyl)-1-nicotinoylpiperazine and addition salts thereof.

8. 4-(4-chloro-2-methylphenyl)-1-nicothinoylpiperazine and addition salts thereof.

9. 4-(5-chloro-2-methylphenyl)-1-nicotinoylpiperazine and addition salts thereof.

10. 4-(4-Acetylphenyl)-1-nicotinoylpiperazine and addition salts thereof.

11. A method for the preparation of a 4-aryl-1-nicotinoylpiperazine compound of formula I according to claim 1, which comprises reacting an N-arylpiperazine of the formula

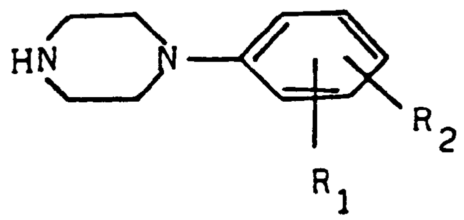

(II)

in which $R_1$ and $R_2$ are defined as indicated above, with a nicotinoyl halide of the formula

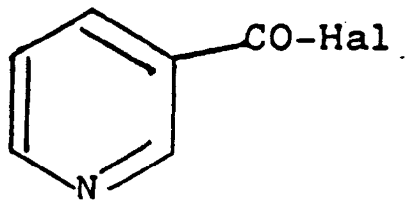

(III)

in which Hal represents F, Cl, or Br, the preferred halogen atom being chlorine.

12. The method according to claim 11 wherein 1 mol of N-arylpiperazine of formula II is reacted with about 1 mol of nicotinoyl halide of formula III for at least 0.25 h at the reflux temperature of the reaction medium.

13. A therapeutic composition which contains, in association with a physiologically acceptable excipient, at least one compound selected from the 4-aryl-1-nicotinoyl-piperazines of formula I according to claim 1 and non-toxic addition salts thereof.

14. Use of a substance belonging to the group comprising the 4-aryl-1-nicotinoylpiperazines of formula I according to claim 1 and non-toxic addition salts thereof in order to obtain a drug for use in therapy vis-a-vis convulsions and especially convulsions of epilectif origin.

**Claims for the following Contracting States : ES, GR**

1. A method for the preparation of a nicotinoylpiperazine compound selected from the group consisting of :
(i) 4-aryl-1-nicotinoylpiperazines of the formula

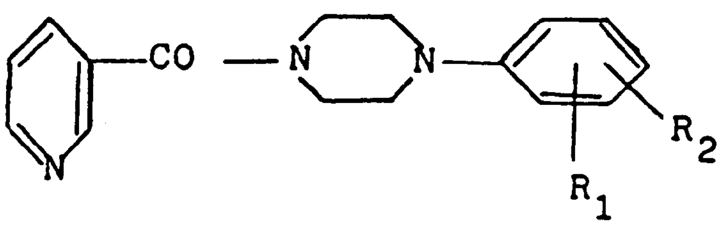

(I)

in which $R_1$ and $R_2$, which can be identical or different, each represent the hydrogen atom, a halogen atom or a $CF_3$, $C_1$-$C_4$-alkyl or $C_1$-$C_4$-alkoxy group ; and
(ii) addition salts thereof ; said method comprising reacting an N-arylpiperazine of the formula

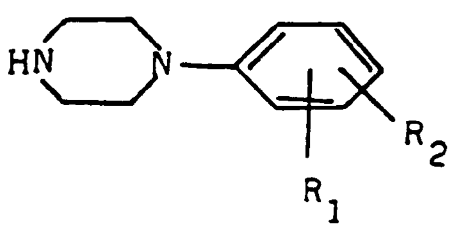

(II)

in which $R_1$ and $R_2$ are defined as indicated above, with a nicotinoyl halide of the formula

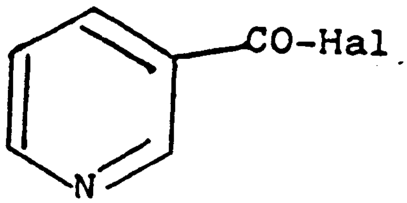

(III)

in which Hal represents F, Cl, or Br, the preferred halogen atom being chlorine.

2. The method according to claim 1 wherein 1 mol of N-arylpiperazine of formula II is reacted with about 1 mol of nicotinoyl halide of formula III for at least 0.25 h at the reflux temperature of the reaction medium.

3. A method according to claim 1, wherein Hal is H.

4. A method according to claim 1, wherein the halogen atom within the definition of $R_1$ and $R_2$ is selected from F, Cl and Br.

5. A method according to claim 1, wherein $R_1$ and $R_2$, which can be identical or different, each represent Cl, F, $CF_3$, $CH_3$, $C_2H_5$, $OCH_3$ or $OC_2H_5$, it being possible, if appropriate, for only one of the groups $R_1$ and $R_2$ to represent the hydrogen atom.

6. A method according to anyone of the claims 1-5 wherein $R_1$ is in the 2-position and $R_2$ is in the 3-,4-,5- or 6-position of the benzene ring.